# EUROPÄISCHE PATENTANMELDUNG

(11) **EP 2 401 959 A1**
(43) Veröffentlichungstag der Anmeldung: **04.01.2012**
(21) Anmeldenummer: 11171800.3
(22) Anmeldetag: 28.06.2011
(51) Int. Cl.: A61B 5/0245, A63B 24/00

(54) **Verfahren und Vorrichtung zur Steuerung von Lastparametern eines Trainingsgerätes**

(30) Priorität: 01.07.2010 DE 102010030837
(71) Anmelder: Schelzig, Nil, 51491 Overath (DE)
(72) Erfinder: Schelzig, Nil, 51491 Overath (DE)
(74) Vertreter: Albrecht, Ralf

(57) **Zusammenfassung**

Die Erfindung betrifft Verfahren zur Steuerung von Lastparametern eines Trainingsgerätes (2) in Abhängigkeit vom Herzpuls einer trainierenden Person, bei dem der Herzpuls der trainierenden Person von einem Herzpulssensor (8) erfasst wird, ein Steuerungsgerät (4) dem Herzpuls entsprechende Pulssignale auf der Basis von gespeicherten Trainingsprogrammen moduliert, und bei dem das Trainingsgerät (2) auf der Basis modulierter Pulssignale (16) einem vorprogrammierten Trainingsablauf folgt, der vorab durch die trainierende Person an einer Steuerungseinheit (6) des Trainingsgerätes (2) angewählt wurde.

## Beschreibung

Die vorliegende Erfindung betrifft ein Verfahren zur Steuerung von Lastparametern eines Trainingsgerätes in Abhängigkeit vom Herzpuls einer trainierenden Person, bei dem der Herzpuls der trainierenden Person von einem Herzpulssensor erfasst wird und bei dem das Trainingsgerät auf der Basis empfangener Pulssignale einem vorprogrammierten Trainingsablauf folgt, der vorab durch die Person an einer Steuerungseinheit des Trainingsgerätes angewählt wurde. Des weiteren betrifft die Erfindung ein Steuerungsgerät, einen Brustgurt und eine Trainingsvorrichtung zur Durchführung des Verfahrens.

Trainingsvorrichtungen der eingangs genannten Art sind bekannt und beispielsweise in der EP 0 650 695 B1 beschrieben. Die aus dieser Druckschrift bekannte Trainingsvorrichtung umfasst ein Trainingsgerät und ein Herzpulsmessgerät.

Bei dem Trainingsgerät handelt es sich um ein Laufband, das geeignet ist, Lastparameter in Abhängigkeit von dem Herzpuls bzw. der Pulsfrequenz einer trainierenden Person während eines Trainingsablaufes zu variieren. Alternativ kann das Trainingsgerät insbesondere ein Fahrradergometer oder ein anderes Fitnessgerät sein, wenn es eine variable Belastungs- bzw. Widerstandssteuerung erlaubt. Dazu umfasst das Trainingsgerät eine Steuerungseinheit, die als eine mikroprozessorgesteuerte Zeitmessungs- und Lastparametersteuerungsvorrichtung ausgebildet und mit einem Empfänger verbunden ist, der geeignet ist, Pulssignale zu empfangen, die der Pulsfrequenz der trainierenden Person entsprechen.

Das Herzpulsmessgerät ist als Burstgurt ausgebildet, das über integrierte Hautelektroden die dem Herzpuls entsprechenden Pulssignale generiert. Alternativ werden insbesondere Ohrclips zum Erfassen des Herzpulses eingesetzt. Der Herzpulssensor ist mit einem Sender verbunden, der die dem Herzpuls entsprechenden Pulssignale an das Trainingsgerät zur Steuerung der Lastparameter überträgt.

Während des Trainings zieht die trainierende Person den Brustgurt an, um den Herzpuls zu erfassen. Die durch die Hautelektroden generierten und dem Herzpuls entsprechenden Pulssignale werden von dem Sender des Brustgurtes an die Empfangseinheit des Trainingsgerätes übertragen. Die Steuerungseinheit des Trainingsgerätes variiert auf der Basis der empfangenen Pulssignale die Lastparameter des Trainingsgerätes.

Außerdem kann die trainierende Person an der Steuerungseinheit des Trainingsgerätes aus einer vom Hersteller begrenzten Anzahl von Übungsszenarien einen Trainingsablauf anwählen. Üblicherweise kann sich die trainierende Person zumindest zwischen einem Ausdauertraining und einem zeitgesteuerten Intervalltraining entscheiden. Wählt die trainierende Person beispielsweise das Ausdauertraining mit einer gewünschten Trainingsherzfrequenz von 130 Pulsschlägen pro Minute aus, so verringert die Steuerungseinheit den Widerstand bzw. die Laufgeschwindigkeit des Laufbandes, wenn die Herzfrequenz der trainierenden Person über die ausgewählte Herzfrequenz von 130 Pulsschlägen pro Minute steigt.

Ein Problem dieser Trainingsvorrichtung besteht darin, dass die Trainingsmöglichkeiten auf die vom Hersteller vorgegebene Anzahl an vorbestimmten Trainingsabfolgen begrenzt sind. Individuelle Übungsszenarien, die an die aktuelle Leistungsfähigkeit der trainierenden Person angepasst sind, können nicht trainiert werden.

Ausgehend von diesem Stand der Technik ist es eine Aufgabe der vorliegenden Erfindung, ein individuelles Training mit herkömmlichen Trainingsgeräten zu ermöglichen.

Zur Lösung dieser Aufgabe schafft die vorliegende Erfindung ein Verfahren zur Steuerung von Lastparametern eines Trainingsgeräts der eingangs genannten Art, bei dem in Abhängigkeit vom Herzpuls einer trainierenden Person, bei dem der Herzpuls einer trainierenden Person von einem Herzpulssensor erfasst wird, ein Steuerungsgerät dem Herzpuls entsprechende Pulssignale auf der Basis von gespeicherten Trainingsprogrammen moduliert und die modulierten Pulssignale an einen Empfänger des Trainingsgerätes überträgt, und bei dem das Trainingsgerät auf der Basis der empfangenen modulierten Pulssignale einem vorprogrammierten Trainingsablauf folgt, der vorab durch die Person an einer Steuerungseinheit des Trainingsgerätes angewählt wurde. Eine vorbestimmte Trainingsabfolge bzw. Trainingsablauf kann beispielsweise ein Ausdauertraining sein, bei dem die trainierende Person eine Herzfrequenz von z.B. 130 Pulsschlägen pro Minute am Trainingsgerät vorwählt. Das Trainingsgerät wird dann die Lastparameter bzw. den Widerstand z.B. eines Fahrradergometers derart variieren, dass stets diese vorgewählte Herzfrequenz von 130 Pulsschlägen pro Minute der trainierenden Person gehalten wird.

Erfindungsgemäß ist nun zwischen den Herzpulssensor und das Trainingsgerät ein Steuerungsgerät geschaltet, auf welchem individualisierte Trainingspläne für eine trainierende Person abgelegt sind. Das Steuerungsgerät ist ausgebildet, um die abgespeicherten individualisierten Trainingspläne den vorgegebenen Trainingsabfolgen des Trainingsgeräts zu überlagern. Dazu setzt es die Pulssignale, die der Herzpulssensor erfasst, in von den Herzpulssignalen abweichende modulierte Pulssignale um. Anschließend gibt das Steuerungsgerät die modulierten Signale an das Trainingsgerät weiter. Mit anderen Worten werden durch das Steuerungsgerät vom tatsächlichen Herzpuls abweichende Pulssignale simuliert und an das Trainingsgerät weitergegeben, um dessen Lastparameter zu steuern. Wenn in dem angegebenen Beispiel das Trainingsgerät bei einer steigenden Herzfrequenz über die zuvor ausgewählte Herzfrequenz von 130 Pulsschlägen hinaus den Widerstand des Fahrradergometers verringern würde, damit die Herzfrequenz der trainierenden Person wieder sinkt, kann das Steuerungsgerät mittels der modulierten Pulssignale dem Trainingsgerät eine gleichbleibende oder sogar sinkende Herzfrequenz simulieren, um so die Last zu halten oder zu steigern. Auf der Basis der modulierten Pulssignale kann auf diese Weise ein handelsübliches Trainingsgerät jedes Übungsszenario, beispielsweise ein Intervalltraining, ein individuell abgestimmtes Kraftausdauertraining oder ein Rennen auf einer Rennbahn, ausführen, wenn ein solches Trainingsprogramm am Steuerungsgerät ausgewählt wurde. Es sollte klar sein, dass das Trainingsgerät alternativ auch ein Laufband, ein Ruderergometer oder ein anderes Fitnessgerät mit steuerbaren Lastparametern sein kann.

Nach einer bevorzugten Ausführungsform des erfindungsgemäßen Verfahrens werden in einem ersten Übertragungsschritt die dem Herzpuls entsprechenden Pulssignale von einem mit dem Herzpulssensor verbundenen Sender an das Steuerungsgerät und in einem zweiten Übertragungsschritt die modulierten Pulssignale von dem Steuerungsgerät an einen Empfänger des Trainingsgerätes oder direkt an die Steuerungseinheit des Trainingsgerätes übertragen. Die Übertragung der modulierten Pulssignale an den Empfänger des Trainingsgerätes ermöglicht, das Steuerungsgerät flexibel und einfach einzusetzen, ohne Eingriffe in eine an sich bekannte Trainingsvorrichtung umfassend ein Herzpulsmessgerät und ein Trainingsgerät der eingangs genannten Art vornehmen zu müssen. Somit kann das Steuerungsgerät beispielsweise am Körper der trainierenden Person mitgeführt werden und selbständig oder auf Anweisung die Verbindung zu den Gegenstellen aufbauen. Durch die Möglichkeit, dass die modulierten Pulssignale auch direkt an die Steuerungseinheit des Trainingsgerätes übertragbar sind, kann das Steuerungsgerät zur Erweiterung des Trainingsgerätes beispielsweise in oder an dem Trainingsgerät vorgesehen sein. Dabei kann die Empfangseinheit des Steuerungsgerätes zum Empfangen der Pulssignale eingesetzt werden, ohne eine Verbindung zwischen dem Steuerungsgerät und dem Empfänger des Trainingsgerätes herstellen zu müssen.

Bei einer alternativen Ausführungsform des erfindungsgemäßen Verfahrens erhält das Steuerungsgerät die dem Herzpuls entsprechenden Pulssignale unmittelbar von dem Herzpulssensor und überträgt die modulierten Pulssignale über einen mit dem Steuerungsgerät verbundenen Sender an einen Empfänger des Trainingsgeräts. Beispielsweise können die modulierten Pulssignale von dem Sender eines Brustgurtes übertragen werden, an dem auch der Herzpulssensor vorgesehen ist. Die unmittelbare Weitergabe der Pulssignale von dem Herzpulssensor an das Steuerungsgerät ermöglicht, beide Bauteile insbesondere in einem an sich bekannten Brustgurt zu integrieren. Somit kann die trainierende Person im Training den Brustgurt zur Erfassung des Herzpulses und zur Übertragung der modulierten Pulssignale verwenden, ohne ein zusätzliches Gerät mitführen zu müssen. Gleichfalls kann das Steuerungsgerät die Pulssignale unmittelbar von dem Herzpulssensor eines Ohrclips erhalten, und mit diesem im Ohrclip integriert sein.

Bei einer zweiten alternativen Ausführungsform ist vorgesehen, dass in einem ersten Übertragungsschritt ein mit dem Herzpulssensor verbundener Sender die dem Herzpuls entsprechenden Pulssignale über einen Empfänger des Trainingsgerätes an das mit dem Empfänger verbundene Steuerungsgerät überträgt und in einem zweiten Übertragungsschritt das Steuerungsgerät die modulierten Pulssignale an die Steuerungseinheit des Trainingsgerätes übermittelt. Dies ermöglicht, dass ein herkömmliches Herzpulsmessgerät, insbesondere ein Brustgurt, ohne bauliche Veränderung genutzt werden kann. Ein mit dem Herzpulssensor verbundener Sender übermittelt in an sich bekannter Weise die dem Herzpuls entsprechenden Pulssignale an den Empfänger des Trainingsgerätes. Mit dem Empfänger ist das Steuerungsgerät verbunden, welches die Pulssignale moduliert, und an die Steuerungseinheit des Trainingsgerätes übermittelt. Somit kann ein herkömmliches Trainingsgerät durch einen einfachen Einbau des Steuerungsgeräts derart erweitert werden, dass ein individuelles Training möglich ist.

In bevorzugter Weise kann zumindest ein Übertragungsschritt drahtlos analog durchgeführt werden. Dies ermöglicht, die bei handelsüblichen Trainingsvorrichtungen verbreiteten analogen Sender und Empfänger verwenden zu können.

Weiterhin kann zumindest ein Übertragungsschritt drahtlos digital durchgeführt werden, insbesondere unter Verwendung der Bluetooth-Technologie. Dadurch können vor allem moderne Trainingsgeräte insbesondere mit einer Bluetooth-Schnittstelle verwendet werden. Darüber hinaus kann durch eine Verwendung unterschiedlicher Übertragungsformen in den zwei Übertragungsschritten ebenfalls verhindert werden, dass die Pulssignale von dem mit dem Herzpulssensor verbundenen Sender direkt an den Empfänger des Trainingsgerätes übertragen werden.

Ebenfalls ist es möglich, dass zumindest ein Übertragungsschritt in kodierter Form erfolgt, insbesondere wenn zwei Übertragungsschritte analog oder zwei Übertragungsschritte digital durchgeführt werden. Durch die Übertragung in kodierter Form kann eine direkte Übertragung der Pulssignale von dem mit dem Herzpulssensor verbundenen Sender an den Empfänger des Trainingsgerätes verhindert werden. Dies gewährleistet, dass die dem Herzpuls entsprechenden Pulssignale an das Steuergerät und nicht an die Steuerungseinheit des Trainingsgerätes übertragen werden.

Alternativ kann zumindest ein Übertragungsschritt mit einem Verbindungskabel durchgeführt werden. Dies ermöglicht die Anbindung insbesondere älterer Trainingsgeräte, die nicht einen Funkempfänger aufweisen. Darüber hinaus ist es auch vorstellbar, den mit dem Herzpulssensor verbundenen Sender mit einem Verbindungskabel an das Steuerungsgerät anzuschließen.

Gemäß einer weiteren Ausgestaltung der vorliegenden Erfindung können die Trainingsprogramme über eine physikalische Schnittstelle in das Steuerungsgerät geladen und dort gespeichert werden. Somit können die Trainingsprogramme auf einem externen System, insbesondere einem Computer, angepasst bzw. erstellt werden und über die physikalische Schnittstelle in das Steuerungsgerät geladen werden. Darüber hinaus können über diese Schnittstelle auch Trainingsprogramme mit anderen Steuerungsgeräten anderer trainierender Personen ausgetauscht werden. Die Schnittstelle kann eine drahtlose Datenübertragung ermöglichen, beispielsweise eine Bluetooth- oder eine Infrarot-Schnittstelle, oder auch Anschlüsse beispielsweise für USB-, Mini-USB- oder Firewire-Datenkabel aufweisen.

Ein weiterer Gegenstand der vorliegenden Erfindung betrifft ein Steuerungsgerät zur Steuerung von Lastparametern eines Trainingsgerätes in Abhängigkeit vom Herzpuls einer trainierenden Person, das eine Empfangseinheit umfasst, die ausgebildet ist, um dem Herzpuls entsprechende Pulssignale zu empfangen, das eine Modulationseinheit aufweist, die die dem Herzpuls entsprechenden Pulssignale auf der Basis von gespeicherten Trainingsprogrammen moduliert, und das eine Sendeeinheit umfasst, die ausgebildet ist, um die modulierten Pulssignale zu übertragen. Es hat sich als besonders vorteilhaft gezeigt, das Steuerungsgerät für die Umsetzung der genannten Verfahren einzusetzen.

In Weiterbildung des erfindungsgemäßen Steuerungsgerätes weist es ein Display und/oder eine Eingabeeinrichtung auf. Somit kann die trainierende Person aus einer Vielzahl individualisierter Trainingsprogramme einen geeigneten Trainingsplan am Display und/oder der Eingabeeinrichtung auswählen. Darüber hinaus ermöglicht es der trainierenden Person, den Datenaustausch über die physikalische Schnittstelle zu steuern.

Des Weiteren betrifft ein Gegenstand der vorliegenden Erfindung einen Brustgurt mit einem Herzpulssensor, einem Sender und einem Steuerungsgerät der oben genannten Art. Somit kann die trainierende Person im Training den Brustgurt zur Erfassung des Herzpulses und zur Übertragung der modulierten Pulssignale verwenden, ohne ein zusätzliches Gerät mitführen zu müssen.

Schließlich betrifft ein weiterer Gegenstand der vorliegenden Erfindung eine Trainingsvorrichtung zur Steuerung von Lastparametern eines Trainingsgerätes in Abhängigkeit vom Herzpuls einer trainierenden Person, umfassend ein Trainingsgerät und einen Herzpulssensor zum Erfassen des Herzpulses der trainierenden Person, wobei das Trainingsgerät einen Empfänger zum Empfangen von dem Herzpuls das Steuergerät in einer Trainingsvorrichtung bestehend aus einem Trainingsgerät und einem Herzpulsmessgerät einzusetzen, die ein Steuerungsgerät der genannten Art aufweist. Es hat sich als besonders zweckmäßig gezeigt, das Steuerungsgerät in der Trainingsvorrichtung, die das Trainingsgerät und den Herzpulssensor umfasst, vorzusehen.

Hinsichtlich weiterer vorteilhafter Ausgestaltungen der Erfindung wird auf die Unteransprüche sowie die nachfolgende Beschreibung von drei Ausführungsbeispielen unter Bezugnahme auf die beiliegende Zeichnung verwiesen. In der Zeichnung zeigt
- Figur 1: eine schematische Ansicht einer Trainingsvorrichtung gemäß einer ersten Ausführungsform der vorliegenden Erfindung,
- Figur 2: eine schematische Ansicht einer Trainingsvorrichtung gemäß einer zweiten Ausführungsform der vorliegenden Erfindung, und
- Figur 3: eine schematische Ansicht einer Trainingsvorrichtung gemäß einer dritten Ausführungsform der vorliegenden Erfindung.

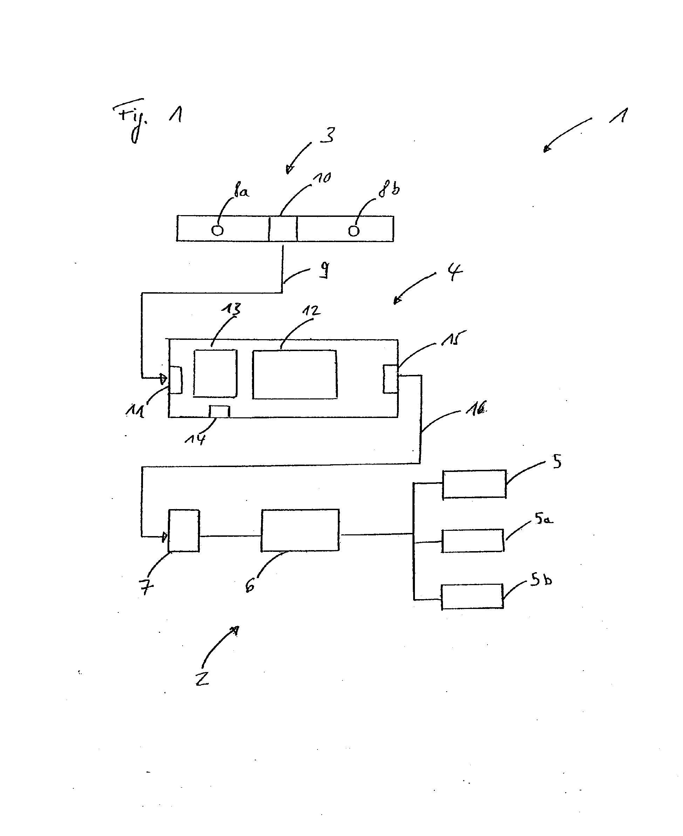

Die Figur 1 zeigt eine Trainingsvorrichtung 1 gemäß einer ersten Ausführungsform der vorliegenden Erfindung. Die Trainingsvorrichtung 1 umfasst ein Trainingsgerät 2, ein Herzpulsmessgerät 3 und ein zwischen Trainingsgerät 2 und Herzpulssmessgerät 3 geschaltetes Steuerungsgerät 4.

Bei dem Trainingsgerät 2 handelt es sich um ein Fahrradergometer 5, das geeignet ist, Lastparameter in Abhängigkeit von dem Herzpuls bzw. der Pulsfrequenz einer trainierenden Person während eines Trainingsablaufes zu variieren. Alternativ kann das Trainingsgerät 2 insbesondere auch ein Laufband, ein Ruderergometer 5a oder ein anderes Fitnessgerät 5b mit steuerbaren Lastparametern sein. Das Trainingsgerät 2 umfasst eine Steuerungseinheit 6, die als eine mikroprozessorgesteuerte Zeitmessungs- und Lastparametersteuerungsvorrichtung ausgebildet ist, welche die Lastparameter des Trainingsgerätes 2 in Abhängigkeit von der Pulsfrequenz der trainierenden Person steuert. Sie ist mit einem Empfänger 7 des Trainingsgerätes 2 verbunden, der geeignet ist, Pulssignale zu empfangen, die der Pulsfrequenz der trainierenden Person entsprechen.

Das Herzpulsmessgerät 3 ist hier in Form eines Brustgurtes ausgebildet. Dieser ist in an sich bekannter Weise als ein elastischer Elektrodengurt mit einem Herzpulssensor 8 ausgebildet. Der Herzpulssensor 8 weist zwei im Brustgurt integrierte Hautelektroden 8a, 8b auf, die auf der Basis des erfassten R-Impulses der trainierenden Person die dem Herzpuls entsprechenden Pulssignale 9 generieren. Mit dem Herzpulssensor 8 ist ein Sender 10 verbundenen, der als Funksender mit geringer Reichweite ausgebildet ist, um die dem Herzpuls entsprechenden Pulssignale 9 zu übertragen. Weiterhin umfasst der Brustgurt 3 eine nicht näher dargestellte Energieversorgung, die üblicherweise in Form einer Lithium-Knopfzelle vorgesehen ist.

In den an sich bekannten Trainingsaufbau bestehend aus Trainingsgerät 2 und Brustgurt 3 ist das Steuerungsgerät 4 zwischen das Trainingsgerät 2 und den Brustgurt 3 geschaltet. Das Steuerungsgerät 4 ist ein portables Gerät, das am Körper einer trainierenden Person mitgeführt werden kann und eine nicht näher dargestellte Energieversorgung umfasst. Es besitzt eine Empfangseinheit 11, die komplementär zum Sender 10 des Brustgurtes 3 ausgebildet ist, um die von dem Sender 10 übertragenen Pulssignale 9 zu empfangen. Mit der Empfangseinheit 11 ist eine Modulationseinheit 12 verbundenen, die geeignet ist, die Pulssignale 9 auf der Basis von auf einem Hardwarespeicher 13 gespeicherten Trainingsprogrammen zu modulieren. Das Steuerungsgerät 4 weist ferner eine integrierte physikalische Schnittstelle 14 auf, die als Bluetooth-Schnittstelle ausgebildet ist, um Trainingsprogramme auf den Hardwarespeicher 13 hoch- bzw. von dem Hardware-speicher 13 wieder runterzuladen. Eine nicht näher dargestellte Eingabeeinrichtung und ein nicht näher dargestelltes Display sind derart ausgebildet, dass die trainierende Person Trainingsprogramme anwählen, die Bluetooth-Schnittstelle 14 steuern und den Ladezustand des Steuerungsgeräts 4 überprüfen kann. Mit der Modulationseinheit 12 ist eine Sendeeinheit 15 verbunden, die derart ausgebildet ist, dass die in der Modulationseinheit 12 modulierten Pulssignale 16 an das Trainingsgerät 2 per Funk übertragbar sind. Ein nicht näher dargestellter Anschluss ist ausgebildet, um ein Netzteil zum Aufladen des internen Akkumulators zu ermöglichen. Dies kann ein USB-Anschluss sein.

Für ein Training mit der Trainingsvorrichtung 1 zieht eine trainierende Person den Brustgurt 3 in bekannter Weise an. Während der Übung auf dem Fahrradergometer 5 erfasst der Herzpulssensor 8 den Herzpuls der trainierenden Person und generiert dem Herzpuls entsprechende Pulssignale 9. Diese werden an den Sender 10 übermittelt, und von dort über die Funk-Schnittstelle an die Empfangseinheit 11 des Steuerungsgeräts 4 übertragen. Dieser erste Übertragungsschritt erfolgt in kodierter Form, um eine direkte Übertragung der Pulssignale 9 an den Empfänger 7 des Trainingsgerätes 2 zu verhindern. Die empfangenen Pulssignale 9 werden an die Modulationseinheit 12 geleitet und dort auf der Basis eines auf dem Hardwarespeicher 13 gespeicherten Trainingsprogramms moduliert.

Bei den Trainingsprogrammen handelt es sich um individualisierte Trainingspläne in digitaler Form, die an die aktuelle Leistungsfähigkeit der trainierenden Person und an deren gewünschte Trainingsziele anpassbar sind. Die trainierende Person kann an einer nicht näher dargestellten Eingabeeinrichtung und an einem Display des Steuerungsgerätes 4 aus einer Vielzahl individualisierter Trainingsprogramme einen geeigneten Trainingsplan auswählen. Außerdem ist die trainierende Person stets in der Lage, vorgefertigte Trainingsprogramme für einen bestimmten Leistungsbereich und ein gewünschtes Trainingsziel aus einer Datenbank im Internet oder eigene mit einer vorgesehenen Computersoftware erstellte Trainingsprogramme mittels eines Computers über die physikalische Schnittstelle 14 auf das Steuerungsgerät 4 zu laden. Darüber hinaus ist es ihr auch möglich, insbesondere über die Bluetooth-Schnittstelle 14 Trainingsprogramme mit anderen Steuerungsgeräten anderer trainierenden Personen auszutauschen.

Die modulierten Pulssignale 16 werden in dem zweiten Übertragungsschritt von der Sendeeinheit 15 an den Empfänger 7 des Trainingsgerätes 2 analog per Funk in nicht-kodierter Form übertragen. An der mit dem Empfänger 7 verbundenen Steuerungseinheit 6 kann die trainierende Person aus einer begrenzten Anzahl von gespeicherten Trainingsabläufen ein Übungsszenario auswählen. Üblicherweise umfassen die Trainingsabläufe einer Steuerungseinheit 6 zumindest ein Ausdauertraining. Hier kann durch die trainierende Person beispielsweise eine Herzfrequenz von 130 Pulsschlägen pro Minute vorgewählt werden. Die Steuerungseinheit 6 wird dann die Lastparameter bzw. den Widerstand des Fahrradergometers 5 variieren, um stets diese vorgewählte Herzfrequenz von 130 Pulsschlägen pro Minute der trainierenden Person zu halten. Diese Eigenschaft macht sich das Steuerungsgerät 4 derart zu nutze, dass der Steuerungseinheit 6 ein abgewandelterer bzw. modulierter Herzpuls 16 mitgeteilt wird, um die Variierung der Lastparameter bzw. des Widerstandes des Fahrradergometers 5 aktiv steuern zu können. Somit kann jedes gewünschte Trainingsprogramm, welches zuvor durch die trainierende Person an dem Steuerungsgerät 4 ausgewählt wurde, dem hier ausgewählten Ausdauertraining überlagert werden. Wo beispielsweise die Steuerungseinheit 6 bei einer steigenden Herzfrequenz über die zuvor ausgewählte Herzfrequenz von 130 Pulsschlägen hinaus den Widerstand des Fahrradergometers 5 verringern würde, damit die Herzfrequenz der trainierenden Person wieder sinkt, kann das Steuerungsgerät 4 mittels der modulierten Pulssignale 16 der Steuerungseinheit 6 eine gleichbleibende, sinkende oder steigende Herzfrequenz, wenn dies gemäß dem ausgewählten Trainingsprogramm erforderlich ist, simulieren. Auf der Basis der modulierten Pulssignale 16 kann nun ein handelsübliches Trainingsgerät 2 jedes Übungsszenario, bspw. ein Intervalltraining, ein individuell abgestimmtes Kraftausdauertraining oder ein Rennen auf einer Rennbahn, ausführen, wenn ein solches Trainingsprogramm am Steuerungsgerät 4 ausgewählt wurde.

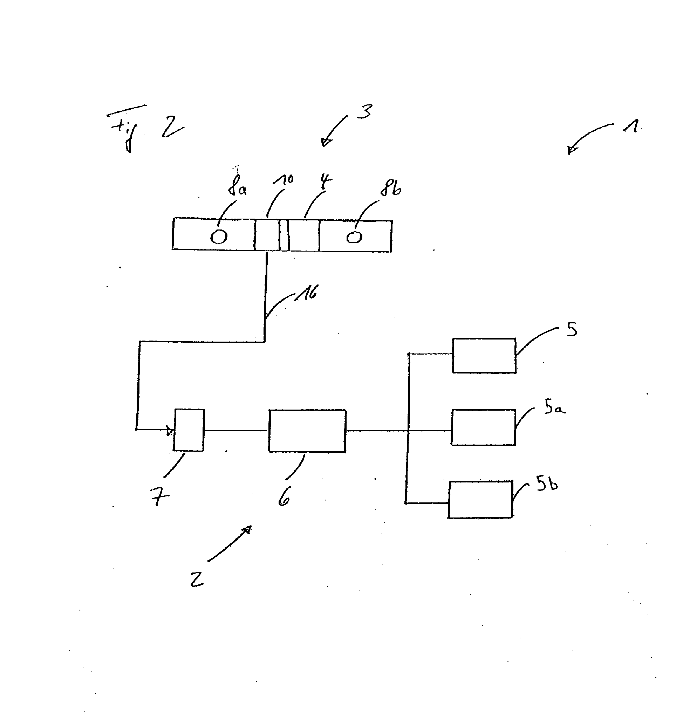

Die Figur 2 zeigt eine Trainingsvorrichtung 17 gemäß einer zweiten Ausführungsform der vorliegenden Erfindung. Die Trainingsvorrichtung 17 umfasst ein Trainingsgerät 2, einen Brustgurt 3 und ein zwischen Trainingsgerät 2 und Herzpulsmessgerät 3 geschaltetes Steuerungsgerät 4, wobei das Steuerungsgerät 4 im Vergleich zur ersten Ausführungsform der vorliegenden Erfindung gemäß Figur 1 in dem Brustgurt 3 integriert ist.

Ein Herzpulssensor 8 des Brustgurtes 3 ist unmittelbar an eine nicht näher dargestellte Empfangseinheit des Steuerungsgerätes 4 angeschlossen. Analog zur ersten Ausführungsform der vorliegenden Erfindung ist das Steuerungsgerät 4 derart ausgebildet, dass es zur Modulation der dem Herzpuls entsprechenden Pulssignale 9 auf der Basis der auf einem Hardwarespeicher gespeicherten Trainingsprogramme geeignet ist. Das Steuerungsgerät 4 weist eine physikalische Schnittstelle auf, die als Bluetooth-Schnittstelle ausgebildet ist, um Trainingsprogramme hoch- und runter zu laden. Ein im Brustgurt 3 integrierter analoger Funksender ist mit dem Steuerungsgerät 4 verbunden, um die von der Modulationseinheit des Steuerungsgerätes 4 modulierten Pulssignale 16 an das Trainingsgerät 2 drahtlos zu übertragen.

Das Trainingsgerät 2 entspricht der ersten Ausführungsform der vorliegenden Erfindung gemäß Figur 1.

Für den Betrieb der Trainingsvorrichtung 17 zieht eine trainierende Person den Brustgurt 3 in bekannter Weise an. Während der Übung auf dem Fahrradergometer 5 erfasst der Herzpulssensor 8 des Brustgurtes 3 den Herzpuls der trainierenden Person und generiert die dem Herzpuls entsprechenden Pulssignale 9. Diese werden durch das mit dem Herzpulssensor verbundene Steuerungsgerät 4 analog zur ersten Ausführungsform der vorliegenden Erfindung moduliert. Die modulierten Pulssignale 16 werden anschließend durch den mit dem Steuerungsgerät 4 verbundenen analogen Sender des Brustgurtes 3 an das Trainingsgerät 2 per Funk übertragen. Dadurch entfällt gegenüber der Trainingsvorrichtung 1 gemäß der Figur 1 der erste drahtlose Übertragungsschritt. Im Weiteren verhält sich das Trainingsgerät 2 analog zur ersten Ausführungsform der Erfindung.

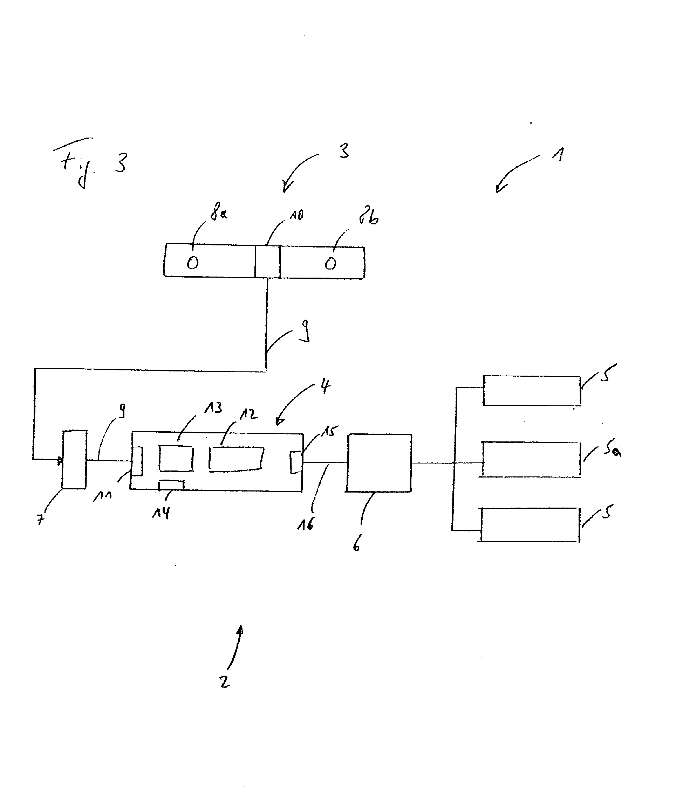

Die Figur 3 zeigt eine Trainingsvorrichtung 17 gemäß einer dritten Ausführungsform der vorliegenden Erfindung. Die Trainingsvorrichtung 17 umfasst ein Trainingsgerät 2 und ein Herzpulsmessgerät 3. Das Trainingsgerät 2 weist einen Empfänger 7, eine Steuerungseinheit 6 und ein Steuerungsgerät 4 auf. Das Steuerungsgerät 4 ist im Trainingsgerät 2 integriert, wobei es eingangsseitig mit dem Empfänger 7 und ausgangsseitig mit der Steuerungseinheit 6 verdrahtet ist.

Analog zur ersten Ausführungsform der vorliegenden Erfindung ist hier ein Herzpulsmessgerät 3 in Form eines Brustgurtes ausgebildet. Ein Sender 10 des Brustgurtes 3 ist geeignet, um dem Herzpuls entsprechende Pulssignale 9 an den Empfänger 7 des Trainingsgerätes zu übertragen. Der Empfänger 7 ist mit dem Steuerungsgerät 4 verdrahtet, um an diesen die Pulssignale 9 zu übermitteln. Die Modulation des Pulssignales 9 entspricht in analoger Weise der ersten Ausführungsform. Schließlich ist das Steuerungsgerät 4 ausgangsseitig mit der Steuerungseinheit 6 verdrahtet, um die modulierten Pulssignale 16 direkt an die Steuerungseinheit 6 zu übermitteln.

Alternativ kann der Sender 10 geeignet sein, die dem Herzpuls entsprechenden Pulssignale 9 direkt an die Empfangseinheit 11 des Steuerungsgerätes 4 zu übertragen. Hier kann auf eine Drahtverbindung des Steuerungsgerätes 4 eingangsseitig mit dem Empfänger 7 verzichtet werden. Ausgangsseitig ist das Steuerungsgerät 4 mit der Steuerungseinheit 6 verdrahtet.

Für den Betrieb der Trainingsvorrichtung 17 zieht eine trainierende Person den Brustgurt 3 in bekannter Weise an. Im Unterschied zur ersten Ausführungsform der vorliegenden Erfindung überträgt der Sender 10 die dem Herzpuls entsprechenden Pulssignale 9 direkt an den Empfänger 7 des Trainingsgerätes. Das mit dem Empfänger verdrahtete Steuerungsgerät 4 erhält die dem Herzpuls entsprechenden Pulssignale 9 und moduliert diese analog zur ersten Ausführungsform der vorliegenden Erfindung. Anschließend überträgt das Steuerungsgerät 4 die modulierten Pulssignale 16 an die über eine Drahtverbindung angeschlossene Steuerungseinheit 6. Die Steuerungseinheit 6 verhält sich analog zur ersten Ausführungsform der vorliegenden Erfindung.

Alternativ überträgt der Sender 10 die dem Herzpuls entsprechenden Pulssignale 9 direkt an die Empfangseinheit 11 des Steuerungsgerätes 4.

## Patentansprüche

1. Verfahren zur Steuerung von Lastparametern eines Trainingsgerätes (2) in Abhängigkeit vom Herzpuls einer trainierenden Person, bei dem der Herzpuls der trainierenden Person von einem Herzpulssensor (8) erfasst wird, ein Steuerungsgerät (4) dem Herzpuls entsprechende Pulssignale auf der Basis von gespeicherten Trainingsprogrammen moduliert, und bei dem das Trainingsgerät (2) auf der Basis modulierter Pulssignale (16) einem vorprogrammierten Trainingsablauf folgt, der vorab durch die trainierende Person an einer Steuerungseinheit (6) des Trainingsgerätes (2) angewählt wurde.

2. Verfahren nach Anspruch 1, **dadurch gekennzeichnet, dass** in einem ersten Übertragungsschritt die dem Herzpuls entsprechenden Pulssignale (9) von einem mit dem Herzpulssensor (8) verbundenen Sender (10) an das Steuerungsgerät (4) und in einem zweiten Übertragungsschritt die modulierten Pulssignale (16) von dem Steuerungsgerät (4) an einen Empfänger (7) des Trainingsgerätes (2) oder direkt an die Steuerungseinheit (6) des Trainingsgerätes (2) übertragen werden.

3. Verfahren nach Anspruch 1, **dadurch gekennzeichnet, dass** das Steuerungsgerät (4) die dem Herzpuls entsprechenden Pulssignale (9) unmittelbar von dem Herzpulssensor (8) erhält und die modulierten Pulssignale (16) über einen mit dem Steuerungsgerät (4) verbundenen Sender (10) an einen Empfänger (7) des Trainingsgerätes (2) überträgt, wobei insbesondere die modulierten Pulssignale (16) von dem Sender (10) eines Brustgurtes (3) übertragen werden, an dem auch der Herzpulssensor (8) vorgesehen ist.

4. Verfahren nach Anspruch 1, **dadurch gekennzeichnet, dass** in einem ersten Übertragungsschritt ein mit dem Herzpulssensor (8) verbundener Sender (10) die dem Herzpuls entsprechenden Pulssignale (9) über einen Empfänger (7) des Trainingsgerätes (2) an das mit dem Empfänger (7) verbundene Steuerungsgerät (4) überträgt und in einem zweiten Übertragungsschritt das Steuerungsgerät (4) die modulierten Pulssignale (16) an die Steuerungseinheit (6) des Trainingsgerätes (2) übermittelt.

5. Verfahren nach einem der vorherigen Ansprüche, **dadurch gekennzeichnet, dass** zumindest ein Übertragungsschritt drahtlos analog durchgeführt wird und/oder dass zumindest ein Übertragungsschritt drahtlos digital durchgeführt wird, insbesondere unter Verwendung der Bluetooth-Technologie und/oder dass zumindest ein Übertragungsschritt in kodierter Form erfolgt, insbesondere wenn zwei Übertragungsschritte analog oder zwei Übertragungsschritte digital durchgeführt werden, und/oder dass zumindest ein Übertragungsschritt mit einem Verbindungskabel durchgeführt wird.

6. Verfahren nach einem der vorherigen Ansprüche, **dadurch gekennzeichnet, dass** die Trainingsprogramme über eine physikalische Schnittstelle (14) in das Steuerungsgerät (4) geladen und dort gespeichert werden.

7. Steuerungsgerät (4) zur Steuerung von Lastparametern eines Trainingsgerätes (2) in Abhängigkeit vom Herzpuls einer trainierenden Person, **dadurch gekennzeichnet, dass** das Steuerungsgerät (4)
- eine Empfangseinheit (11) umfasst, die ausgebildet ist, um dem Herzpuls entsprechende Pulssignale (9) zu empfangen,
- eine Modulationseinheit (12) aufweist, die die dem Herzpuls entsprechenden Pulssignale (9) auf der Basis von gespeicherten Trainingsprogrammen moduliert, und
- eine Sendeeinheit (15) umfasst, die ausgebildet ist, um die modulierten Pulssignale (16) zu übertragen.

8. Steuerungsgerät (4) nach Anspruch 7, **dadurch gekennzeichnet, dass** das Steuerungsgerät (4) einen Hardwarespeicher (13) umfasst, um die Trainingsprogramme zu speichern, wobei insbesondere das Steuerungsgerät (4) eine physikalische Schnittstelle (14) umfasst, um die Trainingsprogramme auf dem Hardwarespeicher (13) des Steuerungsgerätes (4) zu speichern.

9. Steuerungsgerät (4) nach Anspruch 7 oder 8, **dadurch gekennzeichnet, dass** das Steuerungsgerät (4) Anschlüsse für Verbindungskabel aufweist, um dem Herzpuls entsprechende Pulssignale zu empfangen und/oder modulierte Pulssignale über Verbindungskabel zu übertragen.

10. Steuerungsgerät (4) nach einem der Ansprüche 7 bis 9, **dadurch gekennzeichnet, dass** die Sendeeinheit (15) des Steuerungsgeräts (4) ausgebildet ist, um die modulierten Pulssignale (16) analog und/oder digital drahtlos zu übertragen und/oder die Empfangseinheit (11) des Steuerungsgeräts (4) ausgebildet ist, um dem Herzpuls entsprechende Pulssignale analog und/oder digital drahtlos zu empfangen.

11. Steuerungsgerät (4) nach einem der Ansprüche 7 bis 10, **dadurch gekennzeichnet, dass** das Steuerungsgerät (4) ein Display und/oder eine Eingabeeinrichtung aufweist.

12. Brustgurt (3) mit einem Herzpulssensor (8), einem Sender (10) und einem Steuerungsgerät (4) nach einem der Ansprüche 7 bis 11, wobei insbesondere das Steuerungsgerät (4) eingangsseitig mit dem Herzpulssensor (8) und ausgangsseitig mit dem Sender (10) verbunden ist.

13. Trainingsvorrichtung (1) zur Steuerung von Lastparametern eines Trainingsgerätes (2) in Abhängigkeit vom Herzpuls einer trainierenden Person, umfassend ein Trainingsgerät (2) und einen Herzpulssensor (8) zum Erfassen des Herzpulses der trainierenden Person, wobei das Trainingsgerät (2) einen Empfänger (7) zum Empfangen von dem Herzpuls einer trainierenden Person entsprechende Pulssignale und eine damit verbundene Steuerungseinheit (6) zum Steuern der Lastparameter in Abhängigkeit eines Trainingsablaufes aufweist, **dadurch gekennzeichnet, dass** ein Steuerungsgerät (4) nach einem der Ansprüche 7 bis 11 vorgesehen ist.

14. Trainingsvorrichtung (1) nach Anspruch 20, **dadurch gekennzeichnet, dass** die Trainingsvorrichtung (1) einen Sender (10) umfasst, der mit dem Herzpulssensor (8) verbunden ist und insbesondere mit dem Herzpulssensor (8) in einem Brustgurt nach Anspruch 12 vorgesehen ist.

15. Trainingsvorrichtung (1) nach Anspruch 13 oder 14, **dadurch gekennzeichnet, dass** das Steuerungsgerät (4) an dem Trainingsgerät (2) vorgesehen und ausgangsseitig mit der Steuerungseinheit (6) verbunden ist, wobei insbesondere das Steuerungsgerät (4) eingangsseitig mit dem Empfänger (7) verbunden ist.
